# EUROPEAN PATENT APPLICATION

(11) **EP 4 667 945 A1**
(43) Date of publication of application: **24.12.2025**
(21) Application number: 24755841.4
(22) Date of filing: 08.01.2024
(51) Int. Cl.: G01N 35/04

(54) **CHANNEL BRIDGING DEVICE, SAMPLE ANALYZER, AND SAMPLE COLLECTION METHOD**

(30) Priority: 14.02.2023 CN 202310111172
(71) Applicant: Beckman Coulter Laboratory Systems (Suzhou) Co. Ltd., Suzhou, Jiangsu 215126 (CN)
(72) Inventor: ZHAO, Liang, Suzhou, Jiangsu 215126 (CN)
(74) Representative: Maiwald GmbH
(86) International application number: PCT/CN2024/071056
(87) International publication number: WO 2024/169465

(57) **Abstract**

The present application relates to a channel bridging device for sample collection of a sample analyzer, a sample analyzer comprising the channel bridging device, and a method for collecting a sample in a sample analyzer by means of the channel bridging device. The channel bridging device comprises: a body used for carrying a sample holder; and a base, wherein the body is movably attached to the base. The body is configured to be movable between a first position and a second position; in the first position, the body is connected to a sample suction channel and a spare channel to allow for transfer of the sample holder between the sample suction channel and the spare channel; and in the second position, the body is distant from the sample suction channel and the spare channel and allows for movement of a connection device to the first position. The channel bridging device of the present application can free up the connection device during sampling, thereby allowing the connection device to execute other tasks; therefore, the transport capacity of the sample holder is improved, and the sample processing capability and test throughput of the sample analyzer are thus improved.

## Description

### TECHNICAL FIELD

The present application relates to the field of medical technology. Specifically, the present application relates to a channel bridging device for sample collection of a sample analyzer, a sample analyzer including the channel bridging device, and a method for collecting a sample in a sample analyzer by means of the channel bridging device.

### BACKGROUND

The content in this section merely provides background information related to the present disclosure, which may not constitute prior art.

Various analytical detection instruments (also referred as "sample analyzers" herein) for detecting samples in sample containers such as test tubes or cups are indispensable in the field of medicine, particularly clinical medicine and laboratory medicine. The sample analyzer includes a sample loading/unloading unit, a sample processing unit, and a sampling unit located between the sample loading/unloading unit and the sample processing unit.

The sampling unit includes a sampling area and a sampling device with a sampling probe. A sample rack carrying sample containers is transported to the sampling area by means of a shuttle device, and the sample rack is returned to the sample loading/unloading unit by means of the shuttle device for removal after the sampling is completed. During sampling, the shuttle device forms a part of the channel for the movement of the sample rack when the shuttle device is located in the sampling area. At this time, the sample rack can be moved in the sampling area. However, after the shuttle device leaves the sampling area, the channel within the sampling area is interrupted, so the sample rack can no longer be moved in the sampling area.

On the other hand, when the shuttle device is located in the sampling area, the transportation of the sample rack in the sample loading/unloading unit is suspended, leading to a reduced transportation efficiency of sample racks, and thus a decrease in the detection throughput of the sample analyzer. If an additional shuttle device is added to improve the transportation efficiency of sample racks, the structure and operation of the sample analyzer will become more complicated, leading to a significant increase in cost.

### SUMMARY

In view of the above issues of the existing sample analyzers, the inventor of the present application has proposed an improved solution for the sample collection area. In this solution, the shuttle device may be released during sample collection, so that the shuttle device may transport sample racks among various areas in the sample loading/unloading unit during the sampling.

According to one aspect of the present disclosure, there is provided a channel bridging device for sample collection of a sample analyzer. The channel bridging device includes: a body, which is configured for bearing a sample rack; and a base, to which the body is movably attached, wherein the body is configured to be movable between a first position and a second position. In the first position, the body connects a sample aspiration channel with a standby channel to allow for transfer of the sample rack between the sample aspiration channel and the standby channel; and in the second position, the body is away from the sample aspiration channel and the standby channel and allows for movement of a shuttle device to the first position.

The channel bridging device according to the present application may free up the shuttle device during sampling, thereby allowing the shuttle device to perform other tasks, therefore the transport capacity of the sample rack is improved, and the sample processing capacity, analysis efficiency and detection throughput of the sample analyzer are thus improved.

In some embodiments, a guide member for guiding movement of the body is disposed on the base.

In some embodiments, the guide member is provided with a rail, and the body is provided with a groove fitted with the rail.

In some embodiments, the channel bridging device further includes a biasing member for biasing the body to the first position.

In some embodiments, the channel bridging device further includes a stopper for limiting the body in the first position.

In some embodiments, the channel bridging device further includes a sensor for sensing a position of the body.

In some embodiments, the body includes a sample bearing part for loading the sample rack and a connecting part for connecting the sample bearing part to the base.

According to another aspect of the present application, a sample analyzer is provided, including the above channel bridging device and a shuttle device for transferring a sample rack among various areas of the sample analyzer.

In some embodiments, the body of the channel bridging device is configured to move from the first position to the second position under pushing of the shuttle device.

According to another aspect of the present disclosure, a method for collecting a sample in a sample analyzer by the above channel bridging device is provided, including the following steps: moving a shuttle device loaded with a sample rack to the channel bridging device, with the channel bridging device in the first position; moving the channel bridging device to the second position and moving the shuttle device to the first position; moving the sample rack on the shuttle device to the standby channel; and moving the shuttle device away from the first position and moving the channel bridging device to the first position.

In some embodiments, the method further includes: moving the sample rack from the sample aspiration channel to the standby channel by means of the channel bridging device after the sample is aspired.

In some embodiments, the method further includes: moving the channel bridging device to the second position and moving the shuttle device to the first position; moving the sample rack from the standby channel to the shuttle device; and moving the shuttle device away from the first position and moving the channel bridging device to the first position.

In some embodiments, the channel bridging device is pushed by the shuttle device to move from the first position to the second position.

In some embodiments, the channel bridging device is moved from the second position to the first position by a biasing member.

Other application fields will become apparent from the description provided herein. It should be understood that the specific examples and embodiments described in this section are for illustrative purposes only and are not intended to limit the scope of the present disclosure.

### BRIEF DESCRIPTION OF THE DRAWING

Features and advantages of one or several embodiments of the present disclosure will become more easily understood from the following description with reference to the accompanying drawings, in which:
FIG. 1 is a perspective schematic view of a sample analyzer according to an embodiment of the present disclosure, wherein a lid is opened to allow observation of an internal structure of the sample analyzer;
FIG. 2 is a partially enlarged schematic view of the sample analyzer in FIG. 1 viewed from a right side;
FIG. 3 and FIG. 4 are partially enlarged schematic views of a sampling area of the sample analyzer;
FIG. 5a to FIG. 5e illustrate a loading and sampling process of a sample rack during sampling;
FIG. 6 is a perspective schematic view of a channel bridging device according to a first embodiment of the present disclosure;
FIG. 7 is an exploded schematic view of the channel bridging device in FIG. 6; and
FIG. 8 and FIG. 9 are schematic views of a channel bridging device according to a second embodiment of the present disclosure.

### DETAIL DESCRIPTION OF EMBODIMENTS

Exemplary embodiments will now be described more fully with reference to the accompanying drawings. Corresponding reference numerals in the accompanying drawings indicate the same or corresponding components or features throughout.

Exemplary embodiments are provided so that the present disclosure will be exhaustive and will more fully convey the scope to those skilled in the art. Many specific details such as examples of specific components, devices, and methods are set forth to provide a thorough understanding of various embodiments of the present disclosure. It will be apparent to those skilled in the art that the exemplary embodiments may be implemented in many different forms without specific details, and should not be construed as limiting the scope of the present disclosure. In some exemplary embodiments, well-known processes, well-known device structures, and well-known technologies are not described in detail.

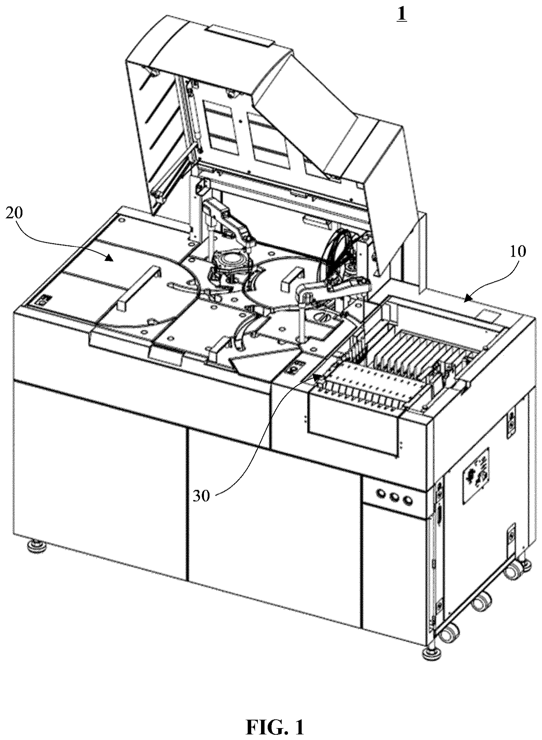

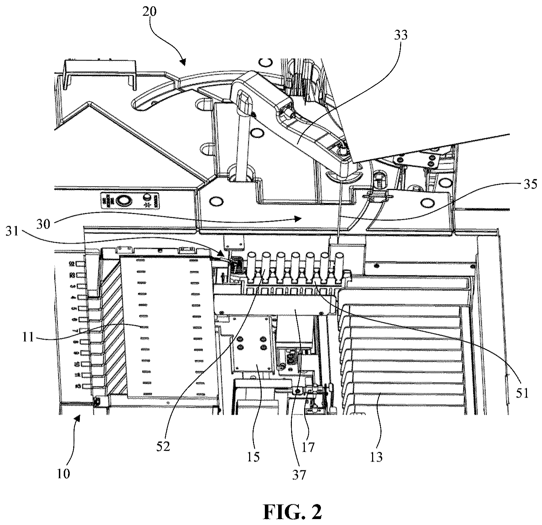

A sample analyzer 1 according to an embodiment of the present disclosure will be described below with reference to FIG. 1 and FIG. 2. It should be understood that the sample analyzer 1 shown in the figures is for illustrative purposes only and should not be limited to the specific examples shown in the figures. The sample analyzer according to the present disclosure may be any suitable sample analyzer for testing or processing biological samples or other chemical samples, for example, a clinical chemistry detector or an immunoassay detector.

FIG. 1 is a perspective schematic view of a sample analyzer 1 according to an embodiment of the present disclosure, wherein a lid is opened to allow observation of an internal structure of the sample analyzer; and FIG. 2 is a partially enlarged schematic view of the sample analyzer 1 in FIG. 1 viewed from a right side. As shown in FIG. 1 and FIG. 2, the sample analyzer 1 includes a sample loading/unloading unit 10, a sample processing unit 20, and a sampling unit 30. The sample loading/unloading unit 10 is configured to load or unload a sample rack 51, on which sample containers 52 containing samples are carried. The sample processing unit 20 is configured to process or detect the sample. The sampling unit 30 is configured to aspirate the sample from the sample container 52 and transfer the sample to the sample processing unit 20 for processing or detection.

The above units of the sample analyzer 1 may be independent of each other, or may be integrated into one piece. The structures of the above units of the sample analyzer 1 may vary depending on the type of the sample analyzer. For example, sample processing units 20 of the same type or different types, as well as sampling units 30 of the same type or different types, may be arranged on both sides of the sample loading/unloading unit 10 respectively.

The sample loading/unloading unit 10 includes a loading/unloading area 11, a buffer area 13, and a transfer area 15. The sample rack 51 may be loaded in or removed from the loading/unloading area 11 by an operator. The shuttle device 17 moves in the transfer area 15 to transport the sample rack 51 among the loading/unloading area 11, the buffer area 13, and the sampling unit 30. The buffer area 13 serves as an area for temporarily placing the sample rack 51 to wait for the next execution process (e.g., detection, unloading, or obtaining detection results).

The sampling unit 30 includes a sampling area 31, a sampling device 33 and a channel bridging device 37. The sampling area 31 is a spatial area where the sample rack 51 is located during sampling. The sampling device 33 has a sampling probe 35. The sampling probe 35 may aspirate the sample from the sample container 52, store the sample, and release the sample when it reaches a predetermined position in the sample processing unit 20. The channel bridging device 37 is configured to be communicated with the individual channels in the sampling area 31 so as to move the sample rack 51.

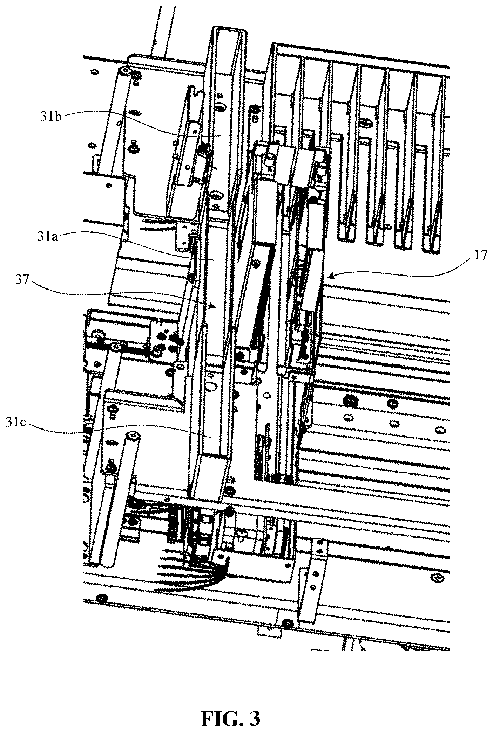

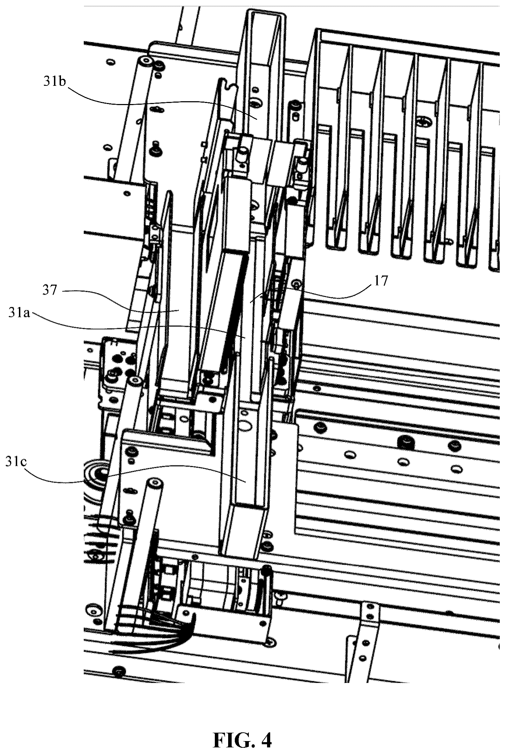

The sampling area 31 will be described below with reference to FIGS. 3 and 4. In order to clearly show the individual channels in the sampling area 31, the sample rack 51 is not shown in FIGS. 3 and 4. As shown in FIGS. 3 and 4, the sampling area 31 includes a sample rack loading/unloading channel 31a, a sample aspiration channel 31b and a standby channel 31c. The sample aspiration channel 31b and the standby channel 31c are on opposite sides of the sample rack loading/unloading channel 31a. In other words, the sample rack loading/unloading channel 31a is communicated with the sample aspiration channel 31b and the standby channel 31c. During loading the sample rack 51 into the sampling area 31 or unloading the sample rack 51 from the sampling area 31, the shuttle device 17 serves as the sample rack loading/unloading channel 31a, as shown in FIG. 4. During periods other than loading and unloading the sample rack 51, the channel bridging device 37 serves as the sample rack loading/unloading channel 31a, as shown in FIG. 3.

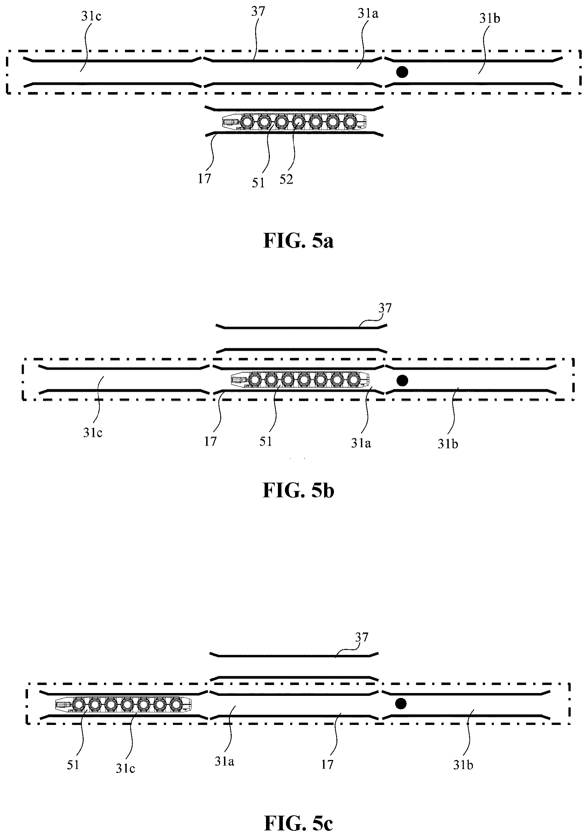

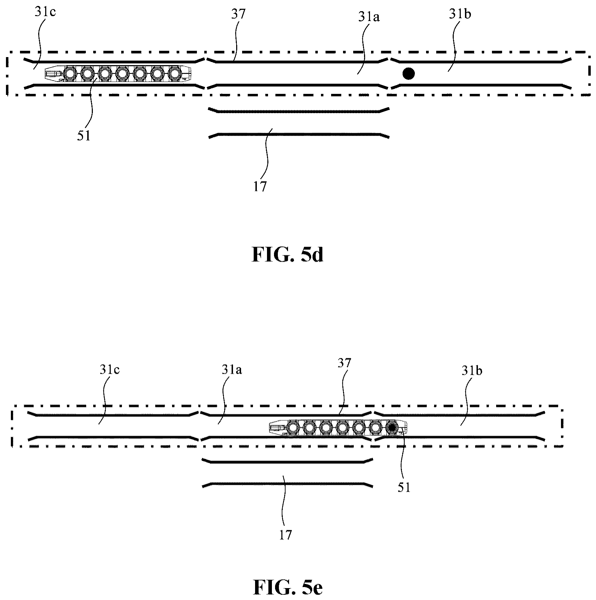

The loading and sampling process of the sample rack performed by the channel bridging device 37 in the sample analyzer 1 will be described below with reference to FIGS. 5a to 5e. When sampling begins, as shown in FIG. 5a, the shuttle device 17 is loaded with the sample rack 51 and moved toward the sampling area 31. At this time, the channel bridging device 37 is in a first position and serves as the sample rack loading/unloading channel 31a for connecting the sample aspiration channel 31b and the standby channel 31c. Then, as shown in FIG. 5b, the channel bridging device 37 moves far away from the sample aspiration channel 31b and the standby channel 31c to a second position, and the shuttle device 17 moves to the first position together with the sample rack 51 and serves as the sample rack loading/unloading channel 31a for connecting the sample aspiration channel 31b and the standby channel 31c. Next, the sample rack 51 is moved from the shuttle device 17 to the standby channel 31c, as shown in FIG. 5c. At this time, the shuttle device 17 may leave the sampling area 31 to perform other tasks. After the shuttle device 17 has left the first position, the channel bridging device 37 returns to the first position to connect the sample aspiration channel 31b and the standby channel 31c, as shown in FIG. 5d. Even without the shuttle device 17, the sample aspiration channel 31b may still be communicated with the standby channel 31c by means of the channel bridging device 37, such that the sample rack 51 may move freely in the sampling area 31. Therefore, the sample rack 51 may move from the standby channel 31c to the sample aspiration channel 31b by means of the channel bridging device 37, as shown in FIG. 5e. At this time, the sample containers may be sequentially sampled by the sampling probe 35 until the sampling is completed.

After the sampling is completed, the unloading process of the sample rack is just the reverse of the loading process the sample rack, that is, the sample rack 51 is moved sequentially through the positions shown in FIG. 5e to FIG. 5a. Specifically, after the sample collection of all sample containers 52 on the sample rack 51 is completed, the sample rack 51 is moved from the sample aspiration channel 31b shown in FIG. 5e to the standby channel 31c shown in FIG. 5d by means of the channel bridging device 37. Then, as shown in FIG. 5c, the channel bridging device 37 is moved from the first position to the second position, and the shuttle device 17 is moved to the first position. At this time, the sample rack 51 may be moved from the standby channel 31c to the shuttle device 17, as shown in FIG. 5b. After the sample rack 51 has been transferred to the shuttle device 17, the shuttle device 17 leaves the sampling area 31 together with the sample rack 51. The channel bridging device 37 may return from the second position to the first position after the shuttle device 17 has left the first position, or the channel bridging device 37 may return to the first position after the shuttle device 17 has loaded another sample rack into the sampling area 31.

It should be understood that the various steps of the above sampling process may be adjusted as desired and are not necessarily limited to the specific examples described herein.

As can be seen from the above description, by providing the channel bridging device 37, the shuttle device 17 may be freed up during the sampling process, thereby allowing the shuttle device 17 to perform other tasks. The shuttle device 17 is solely responsible for loading the sample rack into the sampling area 31 or unloading the sample rack from the sampling area 31. The shuttle device 17 may transfer other sample racks among various areas of the sample loading/unloading unit 10 while the sample is collected. Therefore, the detection efficiency and throughput of the sample analyzer according to the present application may be significantly improved.

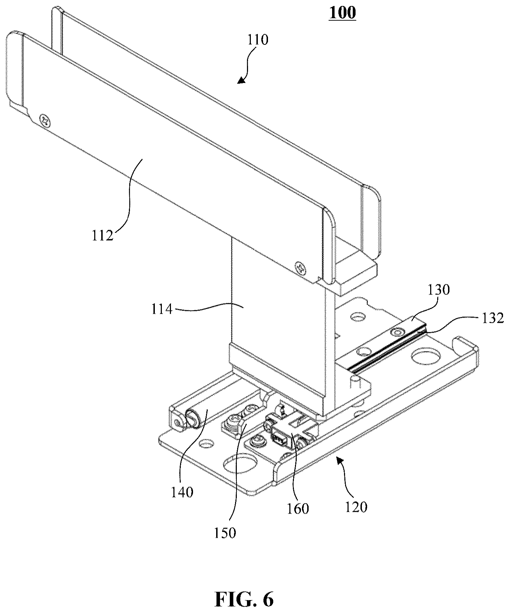

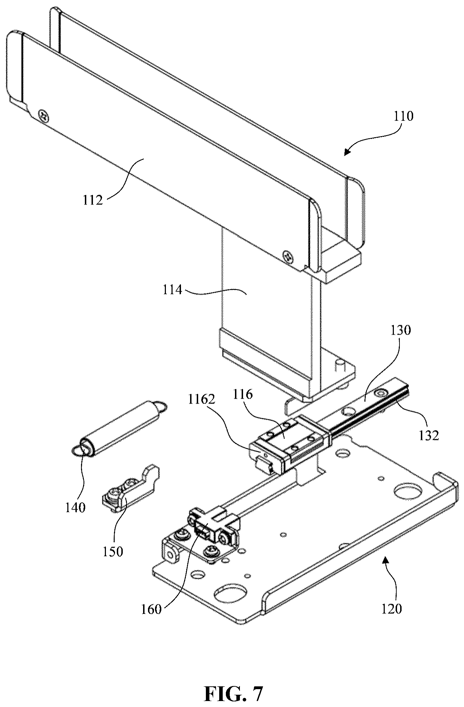

The channel bridging device 100 according to a first embodiment of the present disclosure will be described below with reference to FIGS. 6 and 7. FIG. 6 is a perspective schematic view of the channel bridging device 100 according to the first embodiment of the present disclosure; and FIG. 7 is an exploded schematic view of the channel bridging device in FIG. 6.

As shown in FIGS. 6 and 7, the channel bridging device 100 includes a body 110 and a base 120. The base 120 is fixed in the automatic analyzer 1. The body 110 is movably attached to the base 120, so that the body 110 may be moved between the first position and the second position above. When the body 110 is in the first position as shown in FIGS. 2 and 3, the body 110 may serve as the sample rack loading/unloading channel 31a of the sampling area 31 to bridge the sample aspiration channel 31b and the standby channel 31c. When loading the sample rack to the first position or unloading the sample rack from the first position, the body 110 moves to the second position to allow the shuttle device 17 to move to the first position.

The body 110 includes a sample bearing part 112 for loading a sample rack and a connecting part 114 for connecting the sample bearing part 112 to the base 120. The sample bearing part 112 may form the above sample rack loading/unloading channel 31a. The sample bearing part 112 has a cross-sectional shape matched with that of the sample rack. In the illustrated example, the sample bearing part 112 has a U-shaped cross-section and is configured to bear the sample rack 51. Both ends of the sample bearing part 112 are open to allow communication with the sample aspiration channel 31b and the standby channel 31c, thereby forming a channel in the sampling area 31 for the movement of the sample rack 51. The connecting part 114 has an L-shaped cross-section. The sample bearing part 112 and the connecting part 114 may be integrated into one piece, or they may be separately formed and fixed together.

A guide member 130 for guiding the movement of the body 110 is disposed on the base 120. The guide member 130 has an elongated shape extending in a movement direction of the body 110. The guide member 130 is provided with a rail 132. The body 110 is provided with a groove 1162 fitted with the rail 132. In the illustrated example, the body 110 further includes a guide member 116, and the groove 1162 is provided on a bottom surface of the guide member 116. The guide member 116 is fixed to the connecting part 114. The movement of the body 110 is achieved by sliding the guide member 116 along the guide member 130. In the examples shown in FIGS. 6 and 7, the movement direction of the body 110 is substantially perpendicular to an extension direction of the sample bearing part 112 (i.e., the sample rack loading/unloading channel 31a).

It should be understood that the device for guiding the movement of the body 110 is not necessarily limited to the specific example shown in the figures, but any suitable manner known in the art may be adopted. For example, one of the guide member 116 and the guide member 130 has an elongated slot, and the other of the guide member 116 and the guide member 130 has a pin that is accommodated in the elongated slot and moved along the elongated slot.

The body 110 may be moved from the first position to the second position under the pushing of the shuttle device 17. The channel bridging device 100 may further include a biasing member 140 for biasing the body 110 to the first position. For example, the biasing member 140 may be a spring. One end of the spring is fixed to the base 120, and the other end of the spring is fixed to the guide member 116. When the body 110 is pushed by the shuttle device 17 to move from the first position toward the second position, the spring is stretched and stores energy. When the shuttle device 17 leaves the body 110 (i.e., leaves the first position), the body 110 returns to the first position by the spring.

It should be understood that the device for moving the body 110 is not necessarily limited to the specific examples described herein, but any suitable device known in the art may be adopted. For example, the body 110 may be moved unidirectionally or bidirectionally under the drive of a motor.

The channel bridging device 100 may further include a stopper 150 for limiting the body 110 in the first position. The stopper 150 is fixedly mounted to the base 120. When the channel bridging device 100 is moved to the first position by the biasing member 140, the stopper 150 may prevent the channel bridging device 100 from moving further away from the first position. The stopper 150 is not necessarily limited to the specific example shown in the figures, but may be varied as long as the functions described herein can be achieved.

The channel bridging device 100 may further include a sensor 160 for sensing the position of the body 110. The sensor 160 is, for example, a photoelectric sensor as shown in the figures, which cooperates with a component on the body 110 to sense whether the body 110 is in the first position. For example, when it is sensed that the body 110 is in the first position, the actions shown in FIGS. 5d and 5e may be performed on the sample rack. The sensor 160 may be any suitable device known in the art for detecting position, such as a proximity sensor, a micro switch, etc.

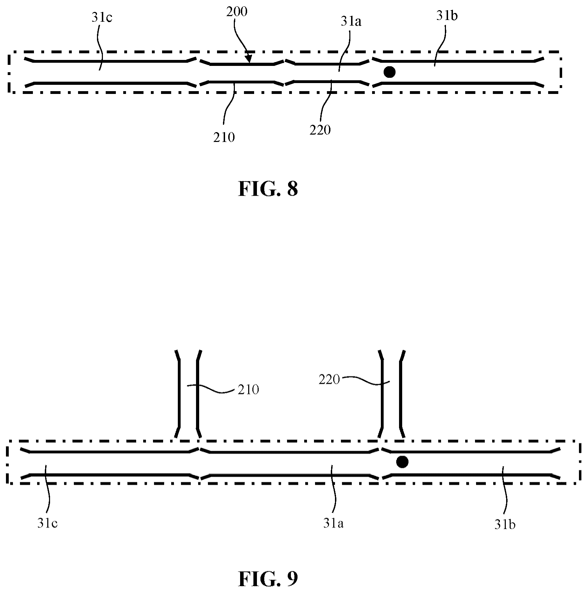

It should be understood that the structure of the body 110 may also be varied as desired and is not necessarily limited to the specific examples shown in the figures. For example, the guide member 116 may be integrated with the connecting part 114. For example, the connecting part 114 may be omitted, and the guide member 116 may be fixed to the sample bearing part 112 or integrated with the sample bearing part 112. In addition, FIGS. 8 and 9 are schematic views of a channel bridging device 200 according to a second embodiment of the present disclosure.

The channel bridging device 200 according to the second embodiment of the present disclosure will be described below with reference to FIGS. 8 and 9. As shown in FIGS. 8 and 9, the channel bridging device 200 includes a first portion 210 and a second portion 220. The first portion 210 and the second portion 220 may be rotated toward or away from each other. The channel bridging device 100 shown in FIGS. 6 and 7 performs linear motion, while the channel bridging device 200 shown in FIGS. 8 and 9 performs rotational motion. In FIG. 8, the first portion 210 and the second portion 220 are both in a first position and aligned with each other to form the sample rack loading/unloading channel 31a. In FIG. 9, the first portion 210 and the second portion 220 rotate away from each other to a second position.

It can be seen that the channel bridging device according to the present disclosure is a device that is independent of the shuttle device and can bridge the sample aspiration channel and the standby channel in the sampling area. The structure and motion form of the channel bridging device should not be limited to the specific examples shown in the accompanying drawings and described herein, but may be varied as long as the functions described herein can be achieved.

Although various embodiments and variations of the present disclosure have been described in detail above, those skilled in the art should understand that the present disclosure is not limited to the specific embodiments and variations described above, but may include other various possible conjunctions and combinations. Other modifications and variations may be implemented by those skilled in the art without departing from the essence and scope of the present disclosure. All these variations and variants shall fall within the scope of the present disclosure. Moreover, all members described herein may be replaced by other technically equivalent members.

## Claims

1. A channel bridging device for sample collection of a sample analyzer, comprising:
a body, which is configured for bearing a sample rack; and
a base, to which the body is movably attached,
wherein the body is configured to be movable between a first position and a second position; in the first position, the body connects a sample aspiration channel with a standby channel to allow for transfer of the sample rack between the sample aspiration channel and the standby channel; and in the second position, the body is away from the sample aspiration channel and the standby channel and allows for movement of a shuttle device to the first position.

2. The channel bridging device according to claim 1, wherein a guide member for guiding movement of the body is disposed on the base.

3. The channel bridging device according to claim 2, wherein the guide member is provided with a rail, and the body is provided with a groove fitted with the rail.

4. The channel bridging device according to claim 1, further comprising a biasing member for biasing the body to the first position.

5. The channel bridging device according to claim 1, further comprising a stopper for limiting the body in the first position.

6. The channel bridging device according to claim 1, further comprising a sensor for sensing a position of the body.

7. The channel bridging device according to claim 1, wherein the body comprises a sample bearing part for loading the sample rack and a connecting part for connecting the sample bearing part to the base.

8. A sample analyzer, comprising the channel bridging device according to any one of claims 1 to 7, and a shuttle device for transferring a sample rack among various areas of the sample analyzer.

9. The sample analyzer according to claim 8, wherein the body of the channel bridging device is configured to move from the first position to the second position under pushing of the shuttle device.

10. A method for collecting a sample in a sample analyzer by the channel bridging device according to claim 1, comprising the following steps:
moving a shuttle device loaded with a sample rack to the channel bridging device, with the channel bridging device in the first position;
moving the channel bridging device to the second position and moving the shuttle device to the first position;
moving the sample rack on the shuttle device to the standby channel; and
moving the shuttle device away from the first position and moving the channel bridging device to the first position.

11. The method according to claim 10, further comprising:
moving the sample rack from the standby channel to the sample aspiration channel by means of the channel bridging device to collect a sample from a sample container loaded on the sample rack.

12. The method according to claim 10, further comprising:
moving the sample rack from the sample aspiration channel to the standby channel by means of the channel bridging device after the sample is aspired.

13. The method according to claim 12, further comprising:
moving the channel bridging device to the second position and moving the shuttle device to the first position;
moving the sample rack from the standby channel to the shuttle device; and
moving the shuttle device away from the first position and moving the channel bridging device to the first position.

14. The method according to any one of claims 10 to 13, wherein the channel bridging device is pushed by the shuttle device to move from the first position to the second position.

15. The method according to any one of claims 10 to 13, wherein the channel bridging device is moved from the second position to the first position by a biasing member.
